# EUROPEAN PATENT APPLICATION

(11) **EP 2 578 191 A1**
(43) Date of publication of application: **10.04.2013**
(21) Application number: 11184412.2
(22) Date of filing: 07.10.2011
(51) Int. Cl.: A61F 9/00, A61M 5/24

(54) **Apparatus for intraocular injection**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is a medicament delivery device comprising a body (1) adapted to accommodate a cartridge (2) containing a medicament (3), and a reservoir (7, 17) coupled to the body (1), the reservoir (7, 17) containing a fluid (13) and a plunger (8, 18). The cartridge (2) is axially movable relative to the body (1) between a retracted position and an extended position. Axial movement of the cartridge (2) from the retracted position to the extended position moves the plunger (8, 18) to expel the fluid (13) from the reservoir (7, 17).

## Description

### Background

A number of vision-threatening disorders or diseases of the eye need to deliver a medicament (drug or proteins or the like) to a posterior segment of the eye by intraocular delivery (more specifically intravitreal delivery), especially when it is useful to deliver high concentrations of medicament. One such technique for intraocular delivery is accomplished by intraocular injection of the medicament or capsules containing the medicament directly into the vitreous body or by locating a device or capsule containing the medicament in the vitreous with a syringe. Such an operation is used in particular for injection of compositions into the vitreous body of the eye in order to treat diseases affecting retina or choroid, or ciliary body or the lens.

After delivery of medicament to the interior, such as the vitreous body, of the eye it is desirable that the point of entry of any medicament delivery closes and heals or seals as quickly and completely as possible after withdrawal of the medicament delivery device or syringe. This sealing is important for the following reasons. This sealing prevents reflux of the delivered medicament. Further, the effects of the operation on the internal eye pressure are reduced. Additionally, sealing assists with the healing of the eye tissue affected generally (e.g. sclera) and therefore prevents infections and other complications. Thus, the risk of infection is reduced.

When carrying out invasive procedures on the eye, e.g. administering medicaments into the vitreous body, it is essential that the surrounding area is rinsed and properly cleaned and disinfected. The current practice is to irrigate with saline and then disinfect with Iodine solution. This involves a number of steps and thus there is a need for improvements.

An additional potential problem with the conjunctival displacement technique is that it is difficult to verify visually that the conjunctiva has, in fact, been displaced prior to administering the injection. This is due to the fact that the conjunctiva is translucent and extremely thin, perhaps only two to four cells thick.

### Summary of the Invention

It is an object of the present invention to provide an apparatus for intraocular injection allowing to clean, disinfect and/or sterilize at least an injection site for an intraocular injection.

In an exemplary embodiment, a medicament delivery device according to the present invention comprises a body adapted to accommodate a cartridge containing a medicament, and a reservoir coupled to the body, the reservoir containing a fluid and a plunger. The cartridge is axially movable relative to the body between a retracted position and an extended position. Axial movement of the cartridge from the retracted position to the extended position moves the plunger to expel the fluid from the reservoir.

In an exemplary embodiment, the delivery device further comprises a needle coupled to the cartridge. In the retracted position, the needle is contained within the body, and in the extended position, the needle is exposed from the body. The needle passes through the reservoir when moving from the retracted position to the extended position.

In an exemplary embodiment, the plunger is coupled to the cartridge.

In an exemplary embodiment, the reservoir includes a foot portion adapted to be placed on an eye. The foot portion has a contour to fit a shape of the eye.

In an exemplary embodiment, the reservoir includes an opening having at least one channel. The channel creates a flow path for the fluid from the reservoir to an exterior of the reservoir. The opening is covered by a cover.

In an exemplary embodiment, the fluid includes at least one of a disinfectant, a solution to promote healing, an analgesic, an antibiotic, a medicament, a marker and an antimicrobial.

In an exemplary embodiment, the delivery device further comprises a ring adapted to align the delivery device on the eye.

In an exemplary embodiment, the delivery device further comprises a finger adapted to displace a conjunctival layer of the eye relative to a sclera layer when the cartridge is moving from the retracted position to the extended position.

The person skilled in the art understands that the present invention is not restricted to the explained possibilities.

The above mentioned advantages as well as other advantages of various aspects of the present invention will become apparent to those of ordinary skill in the art by reading the following detailed description, with appropriate reference to the accompanying drawings.

### Brief Description of the Drawings

Exemplary embodiments of the present invention are described herein with reference to the schematic drawings in which:
Figure 1 shows a sectional view of an exemplary embodiment of a medicament delivery device according to the invention in a retracted position,
Figure 2 shows a sectional view of an exemplary embodiment of a medicament delivery device according to the invention in an advanced position,
Figure 3 shows an enlarged sectional view of a distal end of an exemplary embodiment of a medicament delivery device according to the invention,
Figure 4 shows a view on a distal end of an exemplary embodiment of a medicament delivery device according to the invention,
Figure 5 shows a sectional view of another exemplary embodiment of a medicament delivery device according to the invention in a retracted position,
Figure 6 shows a further sectional view another exemplary embodiment of a medicament delivery device according to the invention,
Figure 7 shows a further sectional view of another exemplary embodiment of a medicament delivery device according to the invention in an advanced position,
Figure 8 shows a sectional view of yet another exemplary embodiment of a medicament delivery device according to the invention in a retracted position,
Figure 9 shows a further sectional view of yet another exemplary embodiment of a medicament delivery device according to the invention,
Figure 10 shows a further sectional view of yet another yet another exemplary embodiment of a medicament delivery device according to the invention, and
Figure 11 shows an isometric view of exemplary embodiments of a medicament delivery device and a separate dispensing tube according to the invention.

### Detailed Description

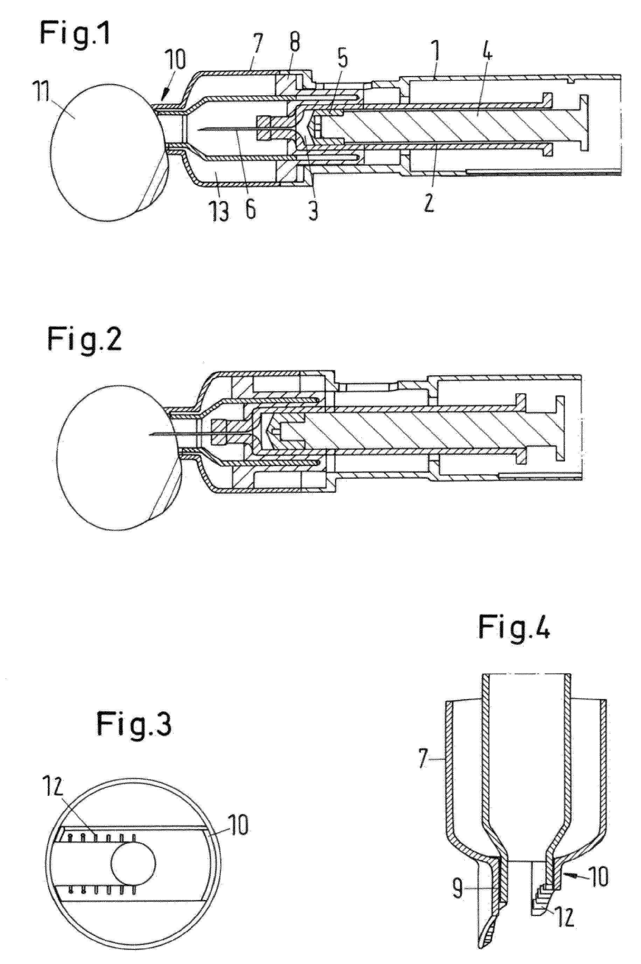

Figures 1 to 4 show exemplary embodiments of a medicament delivery device for an intraocular injection according to the invention. The delivery device has a generally cylindrical outer body or housing 1 which defines a hollow internal space for receiving a cartridge 2 containing at least one medicament 3. Examples of such a medicament are steroids or monoclonal antibodies used to treat macular degeneration, pharmaceuticals, implantable devices, etc. As shown in Figures 1 and 2, the cartridge 2 may have a configuration similar to that of a conventional syringe, e.g., with a plunger 4 acting on a syringe piston 5 to expel the medicament 3 through a needle 6 located at a distal end syringe.

In an exemplary embodiment, the cartridge 2 is received within the body 1 and is axially translatable relative to the body 1. In other words, the cartridge 2 may be moved relative to the body 1 as shown in a retracted position in Figure 1 in which the needle 6 is not exposed (e.g., pre- and post-injection) and an extended position in Figure 2 in which the needle 6 is exposed (e.g., during an injection). Such a relative displacement may either be achieved by advancing the cartridge 2 within the body 1 or by retracting the body 1 relative to the cartridge 2.

Further, a reservoir 7 is coupled to a distal portion of the body 1. A piston 8 is axially moveable within the reservoir 7 in a sealed manner. As shown in Figure 4, a distal part of the reservoir 7 is provided with a discharge opening 9 which is located in a foot portion 10 of the body 1. The foot portion 10 is designed to be placed on the outer surface of an eye 11. For example, the foot portion 10 may be contoured (e.g., circular, semi-circular, etc.) to fit the outer surface of the eye 11. As shown in more detail in Figures 3 and 4, the discharge opening 9 may be in the form of a series of channels 12 which create a flow path for contents of the reservoir 7.

In an exemplary embodiment, the reservoir 7 is prefilled with a fluid 13, e.g., a disinfectant, an antimicrobial, a solution to promote healing, etc. For example, the fluid 13 may be a cleaning and/or sterilizing agent to rinse and clean an injection site on the eye 11 prior to an injection. In addition or as an alternative, the fluid 13 may be a marker fluid, preferably a low viscosity gel which contains a dye or granular pigmentation. This allows marking the injection site such that a visual verification of a movement of the conjunctiva relative to the sclera of the eye 11 is allowed. Further, the fluid 13 or gel may be colored so that the presence and location of the fluid 13 on the eye can be observed.

In the exemplary embodiment shown in Figures 1 and 2, the piston 8 has a distal part which is guided within the reservoir 7 and a proximal part which may be located within the body 1. When a force is applied to the proximal part, the distal part of the piston 8 serves to expel fluid 13 from the reservoir 7. In an exemplary embodiment, the proximal part serves to mechanically slave the piston 8 to the cartridge 2 or another part of the body 1 (e.g., a cartridge carrier) such that the movement of piston 8 is at least partially synchronized with the movement of the cartridge 2 relative to the body 1. In an exemplary embodiment, the movement of the piston 8 is such that the fluid 13 is dispensed on to the surface of the eye 11 after activation of whichever mechanism is used to displace the conjunctiva of the eye 11. In an exemplary embodiment, the reservoir 7 may be telescopically formed on the body 1, and the piston 8 may be an annular member disposed within the reservoir 7 and the body 1.

In use, the exemplary embodiment of the delivery device is placed on the eye 11 as shown in Figure 1 with the foot portion 10 located surrounding an injection site. The cartridge 2 with its needle 6 is then moved relative to the body 1 from the retracted position shown in Figure 1 into the extended position shown in Figure 2. As the movement of the cartridge 2 relative to the body 1 pushes the piston 8 in the distal direction within the reservoir 7, the fluid 13 is expelled through the channels 12 in the foot portion 10. In an exemplary embodiment, the channels 12 are arranged so that the fluid 13 is directed onto the surface of the eye 11 to both sides of the injection site and then across the injection site in the form of a continuous blanket. This may allow sweeping any debris or foreign bodies from the injection site. As an alternative to the embodiment shown in Figures 1 to 4, the shaped channel and foot portion may be designed to direct the fluid 13 into the surface of the eye 11 to one side only of the puncture site and then across the puncture site in the form of a continuous blanket.

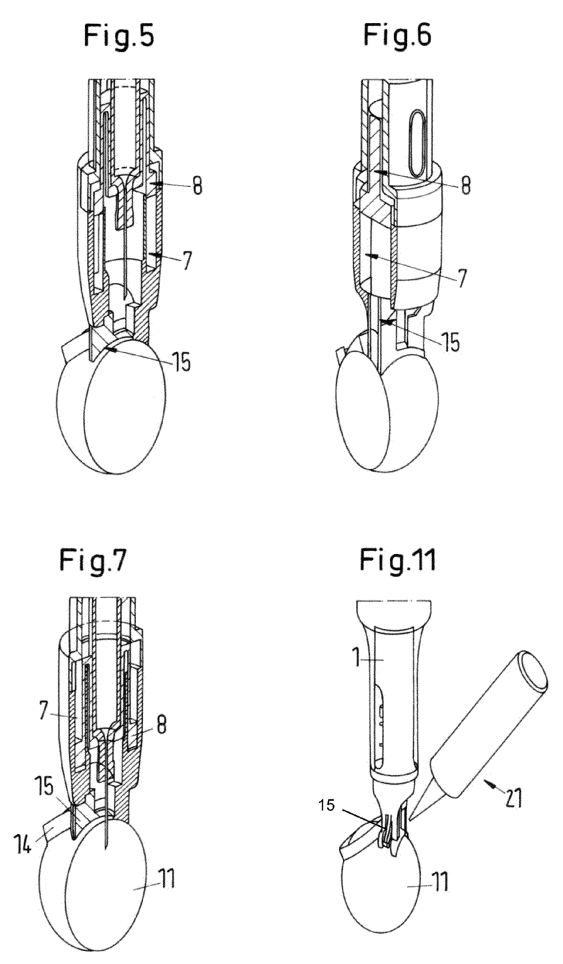

Another exemplary embodiment of the invention is shown in Figures 5 to 7 wherein Figures 5 and 6 show a medicament delivery device with a needle in a retracted position whilst Figure 7 shows the medicament delivery device with the needle in an extended position. The general design of the medicament delivery device is similar to that described above with respect to Figures 1 to 4, i.e. the reservoir 7 is coupled to the body 1, and the body 1 is adapted to receive the cartridge 2 and the needle 6. In this exemplary embodiment, the delivery device includes a ring 14 for positioning the delivery device on the eye 11. Further a finger 15 is provided for displacing the conjunctiva with respect to the sclera prior to the needle 6 piercing the eye 11. Channels 12 are provided which lead from the reservoir 7 to orifices in the region of the finger 15. The use of this exemplary embodiment of the delivery device is similar to that described above.

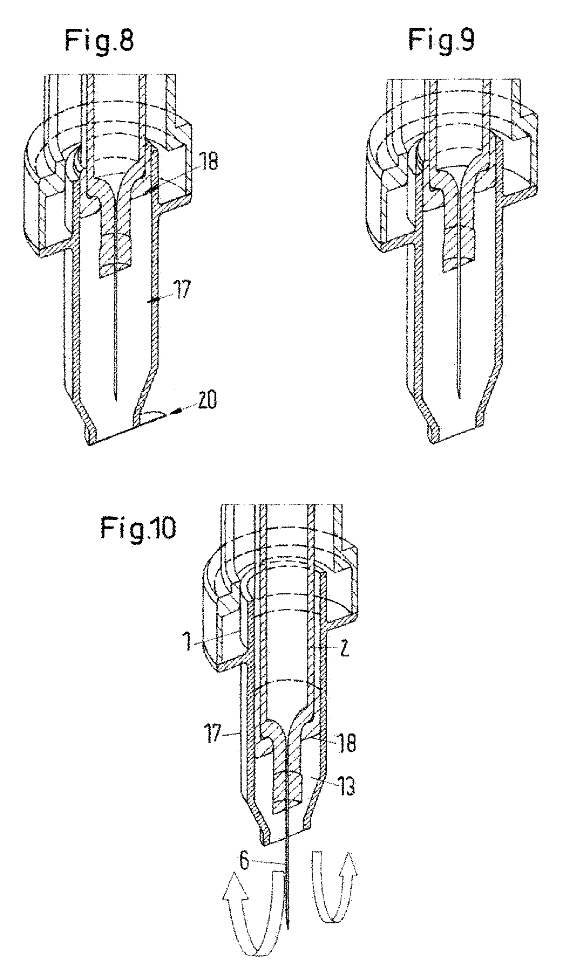

Figures 8 to 10 show a further exemplary embodiment of the present invention, wherein a reservoir 17 is coupled to the distal end of the delivery device, and a distal end of the cartridge 2, including the needle 6, is disposed in the reservoir 17. A piston 18 is provided in the reservoir 17 to expel fluid 13 from reservoir 17 through a distal opening 19. In an exemplary embodiment, the piston 18 has a generally annular form and engages the distal end of the cartridge 2. Thus, the piston 18 is moved in unison with the cartridge 2 if the latter is displaced in the distal direction relative to the body 1.

As can be seen in Figure 8, the opening 19 may be sealed with a cover 20 or similar thereby coincidentally sealing off the needle 6 and the fluid 13. The cover 20 may be removed prior to use as shown in Figure 9. In use of the delivery device, the cartridge 2 is move distally relative to the body 1, and the fluid 13 is expelled from the reservoir 17 by the plunger 18. The fluid 13 covers the puncture site before, during and after the injection process. This is shown in Figure 10. The fluid 13 preferably has a viscosity great enough to remain in place upon and around the puncture site during and after the injection.

An alternative embodiment is shown in Figure 11, a dispensing tube 21 may be used to apply e.g. a marker gel, prior to activation of the medicament delivery device.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament delivery device comprising:
a body (1) adapted to accommodate a cartridge (2) containing a medicament (3), the cartridge (2) being axially movable relative to the body (1) between a retracted position and an extended position; and
a reservoir (7, 17) coupled to the body (1), the reservoir (7, 17) containing a fluid (13) and a plunger (8, 18),
wherein axial movement of the cartridge (2) from the retracted position to the extended position moves the plunger (8, 18) to expel the fluid (13) from the reservoir (7, 17).

2. The medicament delivery device according to claim 1, further comprising:
a needle (6) coupled to the cartridge (2), wherein in the retracted position, the needle (6) is contained within the body (1) and in the extended position, the needle (6) is exposed from the body (1).

3. The medicament delivery device according to claim 2, wherein, the needle (6) passes through the reservoir (7, 17) when moving from the retracted position to the extended position.

4. The medicament delivery device according to any of the preceding claims, wherein the plunger (8, 18) is coupled to the cartridge (2).

5. The medicament delivery device according to any of the preceding claims, wherein the reservoir (7) includes a foot portion (10) adapted to be placed on an eye (11).

6. The medicament delivery device according to claim 5, wherein the foot portion (10) has a contour to fit a shape of the eye (11).

7. The medicament delivery device according to any of the preceding claims, wherein the reservoir (7, 17) includes an opening (9) having at least one channel (12).

8. The medicament delivery device according to claim 7, wherein the channel (12) creates a flow path for the fluid (13) from the reservoir (7) to an exterior of the reservoir (7).

9. The medicament delivery device according to claim 7, wherein the opening (9) is covered by a cover (20).

10. The medicament delivery device according to any of the preceding claims, wherein the fluid (13) includes at least one of a disinfectant, a solution to promote healing, an analgesic, an antibiotic, a medicament, a marker and an antimicrobial.

11. The medicament delivery device according to any of the preceding claims, further comprising:
a ring (14) adapted to align the delivery device on the eye (11).

12. The medicament delivery device according to any of the preceding claims, further comprising:
a finger (15) adapted to displace a conjunctival layer of the eye (11) relative to a sclera layer when the cartridge (2) is moving from the retracted position to the extended position.
